# EUROPEAN PATENT APPLICATION

(11) **EP 1 160 238 A1**
(43) Date of publication of application: **05.12.2001**
(21) Application number: 01113432.7
(22) Date of filing: 01.06.2001
(51) Int. Cl.: C07C 219/04, C07C 213/08, C11D 1/62

(54) **Polyquaternaries from methyldiethanolamine using dibasic acids and fatty acids**

(30) Priority: 01.06.2000 US 208633 P
(71) Applicant: Goldschmidt Chemical Company, Hopewell, VA 23860 (US)
(72) Inventor: Keys, Robert, O., Columbus, OH 43235 (US)
(74) Representative: HOFFMANN - EITLE

(57) **Abstract**

Polyquaternaries which are obtained by reacting at least one alkyldialkanolamine with a mixture of acids comprising at least one fatty acid and at least one dicarboxylic acid are disclosed. The inventive quaternaries have improved properties, such as softness, dispersibility and high biodegradeability; therefore, the inventive esterquats are highly useful as a component in rinse cycle fabric softeners, dryer sheets, hair conditioners, paper debonders and other applications in which a high degree of softness is required.

## Description

### Field of the Invention

The present invention relates to new ester quaternary ammonium compounds ("esterquats") which are obtained by reacting alkyldialkanolamines with a mixture of acids comprising at least one fatty acid and at least one dibasic acid so as to obtain an ester amine; and thereafter quaternizing the resulting ester amine in the presence of an alkylating agent. The esterquats of the present invention exhibit improved dispersibility, softening and fluidity without crosslinking. Moreover, the esterquats of the present invention may be used as a component in a rinse cycle fabric softener, a dryer sheet, a hair conditioner, a two-in-one shampoo, a paper debonder and other applications wherein improved softness is desired.

### Background of the Invention

Esterquats are generally understood by those skilled in the art to be quaternized fatty acid triethanolamine ester salts which are typically used for softening fibers and for conditioning hair. In recent years, esterquats have replaced conventional quaternary ammonium compounds such as distearyl dimethyl ammonium chloride from the market by virtue of their better ecotoxicological compatibility.

Although prior art esterquats have very favorable performance properties and show satisfactory biodegradability and high compatibility with-the skin, consumers are still demanding improved product properties.

U.S. Patent No. 5,880,299 to Ponsati Obiols, et al. describe a new esterquat having improved softening performance which is obtained by reacting trialkanolamines with a mixture of fatty acids and dicarboxylic acids and thereafter quaternizing the resultant ester amine. An optional alkoxylation reaction may be carried out prior to quaternization.

A major drawback with prior art esterquats made from trialkanolamines is that unwanted crosslinking of the various reactants occurs during the quaternization reaction. Crosslinking is undesirable since it typically results in high and unpredictable viscosities.

In view of the above-mentioned drawback with prior art esterquats, there is a continued need for developing new and improved esterquats that do not exhibit any substantial crosslinking during quaternization.

### Summary of the Invention

The present invention relates to esterquats which are useful as a component in rinse cycle fabric softeners, dryer sheets, hair conditioners, paper debonders and other applications wherein improved softness is desired. The esterquats of the present invention are obtained by (a) reacting at least one alkyldialkanolamine with a mixture of acids comprising at least one fatty acid and at least one dicarboxylic acid so as to produce an ester amine; and (b) quaternizing the resultant ester amine produced in step (a) in the presence of an alkylating agent so as to produce an esterquat.

In one optional embodiment of the present invention, the ester amine produced in step (a) is subjected to an optional alkoxylation reaction prior to quaternization. The alkoxylation reaction is carried out utilizing conventional processes that are well known in the art.

The new esterquats produced in the present invention comprise a compound having the following formula: 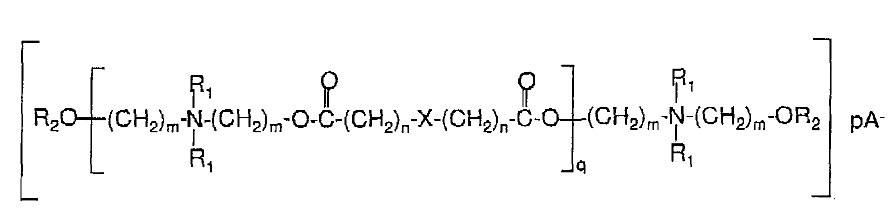 wherein each R₂ is the same or different and is hydrogen or -C(O)R group, each R is the same or different and is a linear or branched, saturated or unsaturated, C₁₁₋₂₁ alkyl; each R₁ is the same or different and is a C₁₋₄ alkyl or hydroxyalkyl; X is a C₁₋₃₆ hydrocarbon which may contain double bonds, aromatic rings, cyclic hydrocarbon groups and branching; each m is the same or different and is an integer from 1 to 6; n is from 1 to 36; q is-from 1 to 20; p is the number of moles of monovalent anion A to provide a net zero charge; and A is a monovalent anion such as chloride, bromide, methyl sulfate, ethyl sulfate, formate, acetate, carbonate, sulfate, nitrate and other like anions.

In one highly preferred embodiment of the present invention, the esterquat is a compound wherein each R₂ is a -C(O)R where R is tallow; each m is 2; each n is 3; X is C₁₋₃₆ hydrocarbon which may contain double bonds, aromatic rings, cyclic hydrocarbon groups and branching; q is 1; each R₁ is methyl; p is 2 and A is chloride.

The esterquats of the present invention which are obtained utilizing an alkyldialkanolamine as a starting reactant exhibit the following properties:
(i) improved softness;
(ii) improved dispersibility;
(iii) improved "clear" products with less solvent;
(iv) less staining;
(v) improved rewetting and adsorption; and
(vi) great flexibility to be used in various applications as a softening agent.

The above properties are not obtained utilizing any prior art esterquat, especially esterquats that are based on trialkanolamines. Moreover, the esterquats of the present invention do not undergo any substantial crosslinking during quaternization, and the inventive esterquats are highly biogradable.

The inventive esterquat of the present invention may be used with other conventional quaternary ammonium compounds, especially conventional esterquats, to provide a concentrate that has improved performance capabilities such as softening, dye transfer, water dispersibility, and etc. In addition to being used with other quaternary ammonium compounds, the esterquats of the present invention may be used with conventional surfactants.

### Description of the Invention

As stated above, the present invention relates to esterquats which are obtained by reacting at least one alkyldialkanolamine with a mixture of acids comprising at least one fatty acid and at least one dicarboxylic acid so as to produce an ester amine; and quaternizing the resultant ester amine in the presence of an alkylating agent.

The esterification of alkyldialkanolamine with a mixture of acids comprising fatty acids and dicarboxylic acids provides a new esterquat which has the following formula: 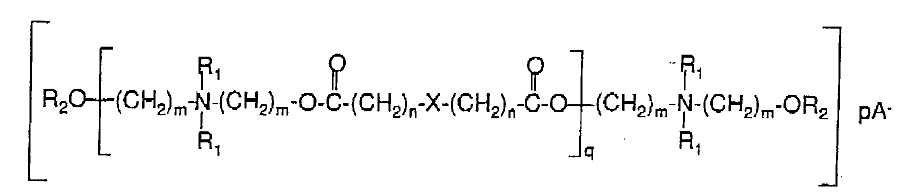 wherein each R₂ is the same or different and is hydrogen or a -C(O)R group, preferably R₂ is a C(O)R group, each R is the same or different and is a linear or branched, saturated or unsaturated, C₁₁₋₂₁ alkyl, preferably a C₁₃₋₁₇ alkyl; each R₁ is the same or different and is a C₁₋₄ alkyl or hydroxy alkyl, preferably R₁ is methyl or ethyl; each m is the same or different and is from 1 to 6, preferably from 2 to 4; each n is the same or different and is from 1 to 36, preferably 2 to 4; q is from 1 to 20, preferably from 1 to 10; X is a C₁₋₃₆, preferably C₁₆₋₁₈ hydrocarbon which may contain double bonds, aromatic rings, cyclic hydrocarbon groups and branching; p is the number of moles of monovalent anion A to provide a net zero charge, preferably 2; and A is a monovalent anion such as chloride, bromide, methyl sulfate, ethyl sulfate, formate, acetate, carbonate, sulfate, nitrate and other like anions, preferably A is chloride.

A highly preferably esterquat of the present invention is one wherein each R₂ is a -C(O)R group where R is tallow; each m is 2; each n is 3; X is C₁₋₃₆ hydrocarbon; q is 1; each R₁ is methyl; p is 2 and A is chloride.

The esterquats of the present invention exhibit surprisingly improved performance properties as compared to esterquats that are made from triethanolamines. In particular, the esterquats of the present invention exhibit improved softening properties which makes the esterquats of the present invention highly suitable for use as a component in rinse cycle fabric softeners. Although the esterquats of the present invention are highly useful in rinse cycle fabric softeners, the inventive esterquats may also be used in other applications in which improved softening is required. For example, the esterquats of the present invention may be used as a component for a hair conditioner, a two-in-one shampoo, a paper debonder, or as a component for a dry sheet formulation.

The present invention is also directed to a process for production of the above defined esterquats in which at least one alkyldialkanolamine is reacted with a mixture of acids comprising at least one fatty acid and at least one dicarboxylic acid to provide an ester amine, and then reacting the ester amine in the presence of an alkylating agent to provide the inventive esterquat.

The term "alkyldialkanolamine" is used herein to denote a compound having the formula 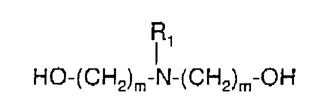 wherein R₁ and m are as defined above. Addition products of the above-identified alkyldialkanolamine with 1 to 10 moles, preferably 2 to 5 moles, of ethylene oxide are also contemplated herein. Illustrative examples of alkyldialkanolamines that can be employed in the present invention include, but are not limited to: methyldimethanolamine, methyldiethanolamine, ethydiethanolamine, propyldiethanolamine, butyldiethanolamine and other like alkyldialkanolamines, Mixtures of one or more of the above mentioned alkyldialkanolamines are also contemplated herein. In one highly preferred embodiment of the present invention, the alkyldialkanolamine is methyldiethanolamine.

Fatty acids that can be employed in the present invention are compounds having the following formula: 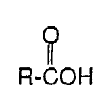 wherein RCO is an aliphatic, linear or branched acyl radical containing 5 to 21 carbon atoms, preferably 1 to 18 carbon atoms, and 0 and/or 1, 2 or 3 double bonds. Illustrative examples of fatty acids that fit the above formula include, but are not limited to: caproic acid, caprylic acid, 2-ethyl hexanoic acid, capric acid, lauric acid, isotridecanoic acid, myristic acid, palmitic acid, palmitoleic acid, stearic acid, isostearic acid, oleic acid, elaidic acid, petroselic acid, linoleic acid, linolenic acid, elaeostearic acid, arachic acid, gadoleic acid, behenic acid, erucic acid and mixtures thereof obtained, for example, by the high pressure hydrolysis of natural fats and oils, in the reduction of aldehydes from Roelen's oxosynthesis or in the dimerization of unsaturated fatty acids. Technical fatty acids (or so-called synthetic fatty acids) containing 12-to 18 carbon atoms such as coconut oil, palm oil, palm kernel oil or tallow fatty acids, preferably in hydrogenated or partially hydrogenated form, are especially preferred in the present invention.

The term "dicarboxylic acid" is used herein to denote a compound having the formula: 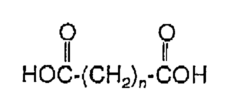 wherein n is as defined above. Typical examples of suitable dicarboxylic acids that may be employed in the present invention include, but are not limited to: succinic acid, maleic acid, adipic acid, glutaric acid, 1,12-dodecanedioic acid and mixtures thereof. Dimer acids from unsaturated feedstock oil like tall oil are also contemplated herein.

In addition to dicarboxylic acids and dimer acids, the present invention also contemplates the use of anhydrides of the above-mentioned dicarboxylic acids. Examples of anhydrides that can be employed in the present invention are succinic anhydride and maleic anhydride. When employed, the dimer acids and anhydrides are used in place of the dicarboxylic acids.

Highly preferably dicarboxylic acids employed in the present invention are succinic acid or adipic acid.

The esterification reaction of the present invention used in forming the ester amine of the inventive esterquats is carried out utilizing techniques that are well known in the art. For example, the esterification reaction may be carried out at a temperature of from about 120°C to about 220°C, preferably from about 130°C to about 170°C, under pressures of from about 0.01 to about 1 bar. The esterification reaction is typically carried out in the presence of a conventional esterification catalyst such as hypophosphite acids and alkali metal salts thereof. In a preferred embodiment of the present invention, sodium hypophosphite is used as the esterification catalyst.

The quantity of the catalyst employed in the esterification reaction varies depending upon the starting reactants used, but typically the catalyst is employed in an amount of from about 0.01 to about 0.1 % by weight, based on starting materials, with quantities from about 0.05 to about 0.07 % by weight being more highly preferred.

In addition to a catalyst, the esterification reaction may take place in the presence of a cocatalyst such as an alkali metal and/or alkaline earth metal borohydride. Examples of suitable cocatalyst that may be employed during the esterification reaction include, but are not limited to: potassium, magnesium and sodium-borohydride. When a cocatalyst is employed, the cocatalyst is used in quantities of from about 50 to about 1000 ppm, based on starting reactants, and more preferably the cocatalyst is used in quantities of from about 100 to about 500 ppm.

The esterification reaction may be carried out on a mixtures of the fatty acids and dicarboxylic acids, or alternatively, the esterification reaction may be carried out with the two components in successive steps.

The fatty acids and the dicarboxylic acids are used in quantities so that a molar ratio of fatty acid to dicarboxylic acid of from about 1:10 to about 10:1 is obtained. More preferably, the molar ratio of fatty acid to dicarboxylic acid in the esterification reaction is from about 1:4 to about 6:1. The alkyldialkanolamines, on the other hand, may be used in a quantity so that the molar ratio of alkyldialkanolamine to acids (combination of all fatty acids and dicarboxylic acids) is from about 1:1.3 to about 1:2.4. A molar ratio of alkyldialkanolamine to acids of from about 1:1.4 to about 1:1.8 is highly preferred.

In an optional embodiment of the present invention, esterquats containing polyalkylene oxide moieties may be produced by two methods. In the first method, ethoxylated alkyldialkanolamines may be used as the starting reactant. In the second method, the ester amine produced in the above described esterification reaction is alkoxylated prior to quaternization. The optional alkoxylation reaction may be carried out utilizing processes well known in the art. Typically, the optional alkoxylation step is carried out in the presence of a basic catalyst and at elevated temperatures. Suitable basic catalysts that may be employed in the optional alkoxylation step include, but are not limited to: alkali metal and alkaline earth metal hydroxides and alcoholates, preferably sodium hydroxide and, more preferably, sodium methanolate. The basic catalysts are normally used in quantities of from about 1 to about 3% by weight, based on the starting reactants, with a quantity of from about 0.5 to about 5% by weight being more highly preferred.

When a basic catalyst is employed, any free hydroxyl groups are primarily alkoxylated. However, if calcined hydrotalcites or hydrotalcites hydrophobicized with fatty acids are used as the basic catalyst, the alkylene oxides are inserted into the ester bonds. This method is preferred when the required alkylene oxide distribution approaches that obtained where alkoxylated alkyldialkanolamines are employed. Ethylene and propylene oxide and mixtures thereof (random or block) may be used as alkylene oxides. The optional alkylation reaction is typically carried out at a temperature of from about 100°C to about 180°C.

The quaternization reaction of the ester amine (or optional alkoylkated ester amine) is carried out utilizing processing techniques that are well known in the art. For example, the quaternization reaction is typically carried out in the presence of an alkylated agent. Although the reaction with the alkylating agent may be carried out in the absence of a solvent, it is preferred to use at least small quantities of water or lower C₁₋₆ alcohol, preferably isopropyl alcohol, for the production of concentrates which have a solids content of at least 80% by weight. The term "low quantities" is used herein to denote an amount of water or lower alcohol of from about 1 to about 15 % by weight, based on the weight of starting reactants.

Suitable alkylating agents include, but are not limited to: alkyl halides such as methyl chloride; dialkyl sulfates such as dimethyl sulfate or diethyl sulfate; or dialkyl carbonates such as dimethyl carbonate or diethyl carbonate.

The esters and alkylating agents are typically used in a molar ratio of 1:0.95 to 1:1.05, i.e., a substantially stoichiometric range. The reaction of ester and alkylating agent is typically carried out at a temperature of from about 40°C to about 80°C, with a temperature of from about 50°C to about 60°C being more preferred. After the alkylation reaction, it is preferred to destroy unreacted alkylating agent by addition of, for example, ammonia, an alkanolamine, an amino acid or an oligopeptide.

The quaternization reaction is normally carried out either in the absence of water or in the presence of a small quantity of solvent such as isopropyl alcohol. However, depending on the particular application of use of the esterquat, the inventive esterquat can be quaternized in the presence of a dispersant or emulsifier.

Suitable dispersants and/or emulsifiers include, but are not limited to: fatty alcohols, technical fatty alcohols, polyols, partial glycerides, and nonionic and ionic surfactants. The weight ratio of ester to dispersant and/or surfactant is from 30:70 to 70:30.

Illustrative examples of fatty alcohols that can be employed as a dispersant and/or emulsifier include, but are not limited to: caproic alcohol, caprylic alcohol, 2-ethylhexyl alcohol, capric alcohol, lauryl alcohol, isotridecyl alcohol, myristyl alcohol, cetyl alcohol, palmitoleyl alcohol, stearyl alcohol, isostrearyl alcohol, oleyl alcohol, elaidyl alcohol, petroselinyl alcohol, linolyl alcohol, linelenyl alcohol, elaeostearyl alcohol, arachyl alcohol, and bassidyl alcohol and technical mixtures thereof obtained, for example, by the high-pressure hydrogenation of technical methyl ester based fats and oils or aldehydes from Roelen's oxosynthesis and as monomer fraction in the dimerization of unsaturated fatty alcohols.

Illustrative examples of technical fatty alcohols include alcohols containing from 12 to 18 carbon atoms such as coconut oil, palm oil, palm kernel oil or tallow fatty alcohol.

Illustrative examples of polyols include, but are not limited to: glycerol, alkylene glycerol such as ethylene glycol, diethylene glycol and propylene glycol, technical oligoglycerol mixtures with a degree of auto-condensation of 1.5 to 10 such as technical diglycerol mixtures with a diglycerol content of 40-50% by weight, methylol compounds such as trimethylol ethane, trimethylol propane, trimethyl butane, petaerythritol and dipentarythritol, lower alkyl (C₁₋₈) glucosides such as methyl and butyl glucosides, sugar alcohols containing 5 to 12 carbon atoms such as sorbitol or mannitol, sugars containing from 5 to 12 carbon atoms such as glucose of sucrose, and amino sugars such as glucamine.

Illustrative partial glycerides include, but are not limited to: monoglycerides and/or diglycerides. Alkyl oligoglucosides, fatty acid-N-alkyl glucamides and adducts of, on average, 1 to 50 moles of ethylene oxide with the fatty alcohols mentioned above are illustrative examples of nonionic surfactants that can be employed in the present invention.

In a preferred embodiment of the present invention, the esterquats of the present invention are used together with one or more conventional quaternary ammonium compounds to provide a concentrate that exhibits improved performance, i.e., softening, dye transfer, water dispersibility, etc. Thus, the esterquats of the present invention, when used in conjunction with one or more conventional quaternary ammonium compounds, provide an unexpected synergy that is heretofore impossible with prior art ester quats that are based on triethanolamines.

Suitable quaternary compounds that can be employed with the esterquat of the present invention include, but are not limited to: the various-quaternary compounds disclosed at Col, 5, line 1-Col. 11, line 64 of U.S. Patent No. 5,674,832 to Keys, et al., the contents of which are incorporated herein by reference. Highly preferred quaternary ammonium compounds that can be used with the inventive esterquat include ester-containing quaternary ammonium compounds such as tallow diquat.

The above quaternary ammonium compounds are well known in the art and from an application point of view the esterquats of the present invention are used in quantities such that a mixture containing a ratio of said esterquats to said conventional quaternary ammonium compounds of from about 10:90 to about 90:10 is obtained.

The esterquats of the present invention that are based on alkyldialkanolamines may be used together with any conventional anionic, nonionic, cationic, zwitterionic or amphoteric surfactants. Mixtures of one or more of the conventional surfactants are also contemplated herein.

Some examples of anionic surfactants that can be employed in the present invention include, but are not limited to: alkylbenzenesulfonates, alkanesulfonates, olefin sulfonates, alkyl ether sulfonates, glycerol ether sulfonates, alpha-methyl ester sulfonates, sulfofatty acids, alkylsulfates, fatty alcohol ether sulfonates, hydroxy mixed ether sulfonates, monoglyceride ether sulfonates, fatty acid amide ether sulfonates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, acyl lactylates, acyl tartrates, acyl glutamates, acyl aspartates, alkyl oligoglucoside sulfates, protein/fatty acid condensates and alkyl ether phosphates.

Examples of nonionic surfactants include, but are not limited to: fatty alcohol polyglycol ethers, alkylphenol polyglycol ethers, fatty acids polyglycol esters, fatty acid amide polyglycol ethers, fatty amine polyglycol ethers, alkoxylated triglycerides, mixed ethers and mixed formals, alk(en)yl oligoglycosides, fatty acid N-alkyl glucamides, protein hydrolyzates, polyol fatty acid esters, sugar esters, sorbitan esters, polysorbates and amine oxides.

Illustrative examples of typically cationic surfactants that the inventive esterquat may be used with include, but are not limited to: quaternary ammonium compounds such as amide amine quats, imidazoline quats and esterquats.

Typical examples of amphoteric or zwitterionic surfactants include, but are not limited to: alkylbetaines, alkylamidobetaines, aminopropionates, aminoglycinates, imidazolinium betaines and sulfobetaines.

The above surfactants are well known in the art and from an application point of view the esterquats of the present invention are used in quantities-such that a mixture containing a ratio of said esterquats to said surfactants of from about 10:90 to about 90:10 is obtained.

In addition to surfactants, the esterquat of the present invention may be used with other conventional ingredients depending upon the final desired application of the esterquats of the present invention. For examples when the esterquat is used as a component for a rinse cycle fabric softener, the rinse cycle fabric softener may include, in addition to the inventive esterquat, conventional detergent builders, enzymes, dye transfer agents, bleaching agents, polymeric soil releasing agents, chelating agents, soil release and anti-deposition agents, optical brighteners, whitening agents, betaines, sultaines, surfactants, hydrotropes, and other like materials that are typically present in rinse cycle fabric softeners or detergents.

When used in other applications such as hair conditioning and paper debonding, the esterquat of the present invention is used in conjunction with conventional ingredients that are typically present in such formulations.

For a complete discussion of ingredients that can be used in fabric softener applications, hair conditioning applications and paper debonders, see coassigned and co-pending U.S. applications Serial Nos. 09/307,521, filed May 7, 1999 and 09/170,623, filed October 13, 1998, the contents of each are incorporated herein by reference.

While the present invention has been particularly shown and described with respect to preferred embodiments thereof, it will be understood by those skilled in the art that the foregoing and other changes in form and detail may be made without departing from the spirit and scope of the present invention. It is therefore intended that the present invention not be limited to the exact forms described and illustrated, but fall within the scope of the present invention.

## Claims

1. An esterquat having the formula 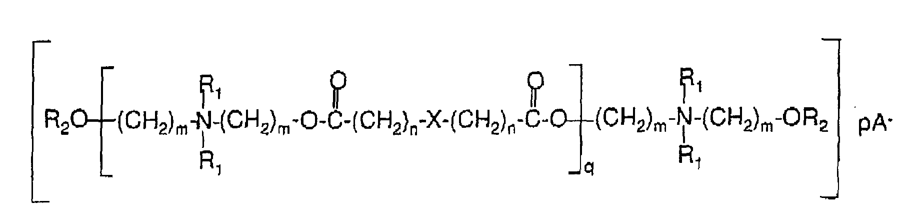 wherein each R₂ is the same or different and is hydrogen or -C(O)R group, each R is the same or different and is a linear or branched, saturated or unsaturated, C₁₁₋₂₁ alkyl; each R₁ is the same or different and is a C₁₋₄ alkyl or hydroxyalkyl; X is a C₁₋₃₆ hydrocarbon; each m is the same or different and is an integer from 1 to 6; each n is the same or different and is from 1 to 36; q is from 1 to 20; p is the number of moles of monovalent anion A to provide a net zero charge; and A is a monovalent anion.

2. The esterquat of Claim 1 wherein each R₂ is a -C(O)R group where R is a C₁₃₋₁₇ alkyl, each R₁ is methyl or ethyl, X is C₁₋₃₆ hydrocarbon; n is from 2 to 4, q is 1-10; and each m is from 2 to 4.

3. The esterquat of Claim 1 wherein said anion, A, is chloride, bromide, methyl sulfate, ethyl sulfate, a formate, an acetate, a carbonate, a sulfate or a nitrate.

4. The esterquat of Claim 2 wherein each R is tallow; each m is 2; n is 3; q is 1; X is X is C₁₋₃₆ hydrocarbon; each R₁ is methyl; p is 2 and A is chloride.

5. A process of preparing an esterquat of Claim 1, wherein said process comprises the steps of:
(a) reacting at least one alkyldialkanolamine with a mixture of acids comprising at least one fatty acid and at least one dicarboxylic acid to produce an ester amine; and
(b) quaternizing the resultant ester amine produced in step (a) in the presence of an alkylating agent so as to produce said esterquat.

6. The process of Claim 5 wherein said ester amine is alkoxylated prior to step (b).

7. The process of Claim 1 wherein said alkyldialkanolamine is a compound having the formula: 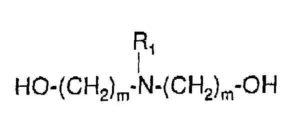 wherein R₁ is a C₁₋₄ alkyl and m is from 1 to 6, or addition products thereof with 1 to 10 moles of ethylene oxide

8. The process of Claim 7 wherein said alkyldialkanolamine is methyldiethanolamine.

9. The process of Claim 5 wherein said fatty acid has the formula: 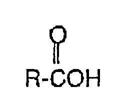 wherein RCO is an aliphatic, linear or branched acyl radical containing 5 to 21 carbon atoms, and from 0, 1, 2 or 3 double bonds.

10. The process of Claim 9 wherein said fatty acid is tallow fatty acid.

11. The process of Claim 5 wherein said dicarboxylic acid has the formula: 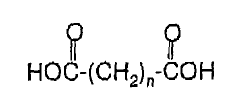 wherein n is from 1 to 36.

12. The process of Claim 11 wherein said dicarboxylic acid is succinic acid or adipic acid.

13. The process of Claim 5 wherein a dimer acid or an anhydride of the said dicarboxylic acid is employed instead of said dicarboxylic acid.

14. The process of Claim 5 wherein said mixture of fatty acids and dicarboxylic acids is present in a molar ratio of from about 1:10 to about 10:1.

15. The process of Claim 5 wherein said alkyldialkanolamine and said mixture of fatty acids and said dicarboxylic acids are present in a molar ratio of from about 1:1.3 to about 1:2.4.

16. The process of Claim 5 wherein said ester salt is formed in the presence of hypophosphorous acid or an alkali metal salt thereof.

17. The process of Claim 5 wherein said alkylating agent is an alkyl halide, a dialkyl sulfate or a dialkyl carbonate.

18. The process of Claim 5 wherein step (b) is carried out in the presence of a dispersant or emulsifier.

19. The process of Claim 18 wherein said dispersant or emulsifier is a fatty alcohol, polyol, partial glyceride, anionic surfactant or nonionic surfactant.

20. A composition of matter comprising at least the esterquat of Claim 1 therein.

21. The composition of matter of Claim 20 further comprising an anionic surfactant, a nonionic surfactant, amphoteric surfactant, zwitterionic surfactant or any mixtures and combinations thereof.

22. The composition of matter of Claim 20 further comprising one or more quaternary ammonium compounds.
